# EUROPEAN PATENT APPLICATION

(11) **EP 3 992 094 A1**
(43) Date of publication of application: **04.05.2022**
(21) Application number: 20853839.7
(22) Date of filing: 13.03.2020
(51) Int. Cl.: B65B 55/04, B65B 7/16, B65B 61/20, A61B 5/145, A61L 2/20, A61L 2/14, B65D 65/42

(54) **STERILIZATION PACKAGING METHOD FOR BODY-ATTACHABLE BIOMETRIC MONITORING DEVICE**

(30) Priority: 19.08.2019 KR 20190101001
(71) Applicant: i-Sens, Inc., Seoul 06646 (KR)
(72) Inventor: CHOI, Hyun Ho, Seoul 06646 (KR); CHAE, Kyung Chul, Seoul 06646 (KR); KANG, Young Jea, Seoul 06646 (KR)
(74) Representative: BCKIP
(86) International application number: PCT/KR2020/003522
(87) International publication number: WO 2021/033864

(57) **Abstract**

The present disclosure relates to a sterilization packaging method for a body-attachable biometric monitoring device. Provided is a sterilization packaging method for a body-attachable biometric monitoring device, the method in which a sterilization packaging process can be performed in a convenient and simple manner, further contamination which can occur during a sterilization process is prevented, damages due to moisture and the like after the packaging is complete are also prevented and thus complete sterilization packaging can be performed, in order to separately perform a sterilization packaging step in two steps, primary packaging is performed for a sterilizable state and then sterilization is performed, secondary packaging is performed to maintain airtightness for a primary packaged article which has been sterilized and thus penetration by an external contaminant and the like during the sterilization and packaging processes is prevented to prevent damages to a biometric monitoring device, a separate adsorbent is positioned outside the primary packaged article and then the secondary packaging is performed and thus functional degradation of the adsorbent during the sterilization process is prevented to enable inner packaging of the adsorbent in an excellent functional state, and a moisture removal function can be performed stably to enable a complete sterilization packaging.

## Description

### TECHNICAL FIELD

The present disclosure is related to a sterilization packaging method for a body-attachable-type biometric monitoring device. In more detail, some exemplary embodiments of the present disclosure relate to a sterilization packaging method for a body-attachable-type biometric monitoring device which can perform a sterilization packaging process in a convenient and simple way, and can prevent additional contamination occurring during a sterile process as well as damage caused by moisture after the packaging is completed, thereby performing sterilization packaging in a more perfect way.

### BACKGROUND

Recently, in modern society, due to the development of biotechnology and information and communication technology, devices for monitoring specific biometric information by attaching to a human body have been being actively developed. Such a biometric information monitoring devices are recognized as an important means for preventing various diseases and avoiding emergency situations due to diseases, because it is possible to eliminate the inconvenience of periodic measurement of biometric information performed manually by a user.

A typical example of a body-attachable-type biometric information monitoring device is a continuous blood glucose measurement device. The continuous blood glucose measurement device is a device that automatically measures blood glucose information from body fluid at regular intervals by inserting a sensor into the human body, and is a very useful device for diabetic patients.

Diabetes occurs because an absolute or relatively insufficient amount of insulin produced by the pancreas due to various reasons such as obesity, stress, wrong eating habits, autoimmunity, and so on causes to be out of balance of glucose in the blood, and in order to diagnose diabetes and manage it not to develop into complications, systematic blood glucose measurement and treatment must be carried out in parallel.

For diabetes patients as well as people having higher than normal blood glucose, even though diabetes has not yet developed, medical device manufacturers offer a variety of blood glucose meters to measure blood glucose levels at home.

Glucose measuring devices may be categorized into a single time measurement type measuring a blood glucose level and collecting blood from a fingertip by a user every single time and a continuous measurement type attaching a glucose monitoring device to the belly or an arm of the user and continuously measuring blood glucose levels.

Diabetics patients generally experience hyperglycemia and hypoglycemia, an emergency may occur in the hypoglycemic conditions, and the patients may become unconscious or die if a hypoglycemic condition lasts for an extended period of time without the supply of sugar. Accordingly, although rapid discovery of the hypoglycemic condition is critically important for diabetics, blood-collecting type glucose monitoring devices intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

A continuous glucose monitoring device includes a sensor module attached to the skin of the human body and measuring a blood glucose level by extracting body fluid, a transmitter transmitting the blood glucose level measured by the sensor module to a terminal, the terminal outputting the received blood glucose level, and any other appropriate component. The sensor module includes a needle-shaped sensor probe for insertion into subcutaneous fat to extract interstitial fluid and any other appropriate component. A separate applicator for attaching the sensor module to the body is used.

Those continuous glucose monitoring devices are manufactured to have a wide variety of types depending on their manufacturers, and are used in a variety of methods. However, the most of the continuous glucose monitoring devices are manufactured and distributed as a type that a one-time use sensor module is attached to the human body using an applicator, and an adhesive tape is attached to a bottom surface of an outside housing of the sensor module so that the sensor module can be attached to the human body. According to such a structure, if the sensor module is insertedly attached to the body skin, a state that the sensor module is attached to the human body is maintained by the adhesive tape, and the blood glucose is continuously measured in this state.

Because such a continuous blood glucose monitoring device includes a body attachable unit such as a sensor module, complete seal in a state of being sterilizedly packaged for contamination prevention should be maintained until the user opens it for use.

In general, chemical sterilization is mainly used as a sterilization method for specific products such as medical devices, and if a specific product is chemically sterilized and then sealed and packaged with a wrapper of gas non-permeable material, the product may be contaminated during this process. Therefore, in order to sterilize and package a body-attachable-type biometric monitoring device such as a continuous blood glucose monitoring device, a high skill level of operators or high-precision sterile packaging equipment is required to prevent contamination of the product in the process of sterilizing the product and the process of sealingly packaging the product, and accordingly there is a problem in that a significant economic cost is needed.

### SUMMARY

### Technical Problem

The present disclosure is invented to solve problems in conventional technique, and the purpose of the present disclosure is for providing a sterilization packaging method for a body-attachable-type biometric monitoring device which can perform a sterilization packaging process in a convenient and simple way, and can prevent additional contamination occurring during a sterile process as well as damage caused by moisture after the packaging is completed, thereby performing sterilization packaging in a more perfect way.

Another purpose of the present disclosure is for providing a sterilization packaging method for a body-attachable-type biometric monitoring device which can maintain non-permeability by performing two separate steps for sterilization packaging which are a first step of performing first-packaging and sterilization to become in a state that sterilization process can be processed, and a second step of second-packaging the sterilized first package to maintain non-permeability, thereby preventing the penetration of the outside contamination substance which may occur during the sterilization and packaging processes, and is capable of performing sterilization packaging of the biometric monitoring device in a convenient and simple way.

Still another purpose of the present disclosure is for providing a sterilization packaging method for a body-attachable-type biometric monitoring device in which, by performing second-packaging after an absorbent is arranged on an outside of a first package, the deterioration of the function of the absorbent during the sterilization process can be prevented and the packaging of the absorbent inside the package can be performed in a good performance state, and, because the function of removing moisture in the inside space of the first wrapper depending on the characteristics of the first wrapper can be stably performed, more perfect sterilization packaging can be performed.

### Solution to Problem

According to an embodiment of the present disclosure, a sterilization packing method for a body-attachable-type biometric monitoring device comprises: an insertion step of inserting the body-attachable-type biometric monitoring device into a storage chamber; a first packaging step of forming a first package by sealing the storage chamber by a first wrapper of gas permeable material in a state that the body-attachable-type biometric monitoring device is inserted in the storage chamber; a sterile step of sterilizing the first package formed through the first packaging step by a chemical sterilization process; and a second packaging step of forming a second package by sealing an outside of the first package by a second wrapper, wherein the second wrapper comprises non-permeable material having gas non-permeable character.

In this embodiment, the sterilization packing method may further comprise an absorbent arrangement step of arranging an absorbent at an outside surface of the first wrapper after the sterile step, wherein the second packaging step comprises forming the second package in a form of accommodating the absorbent, arranged through the absorbent arrangement step, in an inside of the second wrapper.

Additionally, the storage chamber is formed at an inside space of a package case with a container shape of which one side is open, and the first packaging step comprises sealingly coupling the first wrapper to the open one side of the package case in a state that the body-attachable-type biometric monitoring device is inserted in the storage chamber of the package case.

Further, a second attachment surface is formed at an outmost edge portion of the package case facing the open one side, a first attachment surface is formed along an outer edge of a stepped structure at a position away from the second attachment surface in an inside direction of the package case, and at the first packaging step, an outmost edge portion of the first wrapper is sealingly coupled to the first attachment surface.

In addition, at the second packaging step, the second wrapper is sealingly coupled to the second attachment surface.

Additionally, at the absorbent arrangement step, the absorbent is arranged on an outside surface of the first wrapper coupled to the first attachment surface of the package case.

Further, the body-attachable-type biometric monitoring device is configured to be attachable to a human body and periodically measure blood glucose.

In addition, at the sterile step, the chemical sterilization process is a sterilization method using at least one of EO gas, CD gas, low temperature plasma, and steam.

Additionally, the second wrapper comprises gas non-permeable material of which one surface is coated with a metal coating layer.

Meanwhile, according to an embodiment of the present disclosure, the body-attachable-type biometric monitoring device is sterilizingly packaged by the sterilization packing method described above.

### Advantageous Effects of Invention

According to the present disclosure, there is an advantageous technical effect of performing a sterilization packaging process in a convenient and simple way, and preventing additional contamination occurring during a sterile process as well as damage caused by moisture after the packaging is completed, thereby performing sterilization packaging in a more perfect way

Additionally, there is an advantageous technical effect of maintaining non-permeability by performing two separate steps for sterilization packaging which are a first step of performing first-packaging and sterilization to become in a state that sterilization process can be processed, and a second step of second-packaging the sterilized first package to maintain non-permeability, thereby preventing the penetration of the outside contamination substance which may occur during the sterilization and packaging processes, and being capable of performing sterilization packaging of the biometric monitoring device in a convenient and simple way.

Further, there is an advantageous technical effect in that, by performing second-packaging after an absorbent is arranged on an outside of a first package, the deterioration of the function of the absorbent during the sterilization process can be prevented and the packaging of the absorbent inside the package can be performed in a good performance state, and, because the function of removing moisture in the inside space of the first wrapper depending on the characteristics of the first wrapper can be stably performed, more perfect sterilization packaging can be performed.

### BRIEF DESCRIPTION OF THE DRAWINGS

FIGS. 1 and 2 are flowcharts for schematically illustrating a sterilization packaging method for a body-attachable-type biometric monitoring device according to an operation flow according to an embodiment of the present disclosure,
FIG. 3 is an exploded perspective view of schematically illustrating a sterilization package structure of a continuous blood glucose monitoring device according to an embodiment of the present disclosure,
FIG. 4 is figures for schematically illustrating a sterilization packaging method for a continuous blood glucose monitoring device according to an operation flow according to an embodiment of the present disclosure,
FIG. 5 is a figure for schematically illustrating a basic system of a continuous blood glucose monitoring device according to an embodiment of the present disclosure,
FIG. 6 is a figure for schematically illustrating a structure of an applicator of a continuous blood glucose monitoring device according to an embodiment of the present disclosure, and
FIG. 7 is a figure for schematically illustrating a configuration of a body attachable unit of a continuous blood glucose monitoring device according to an embodiment of the present disclosure.

### DETAILED DESCRIPTION OF EMBODIMENTS

Hereinafter, embodiments of the present disclosure will be described in detail with reference to the accompanying drawings. Throughout this document, reference should be made to the drawings, in which the same reference numerals and symbols will be used to designate the same or like components. Additionally, in the following description of the present disclosure, detailed descriptions of known functions and components incorporated herein will be omitted in the case that the subject matter of the present disclosure may be rendered unclear thereby.

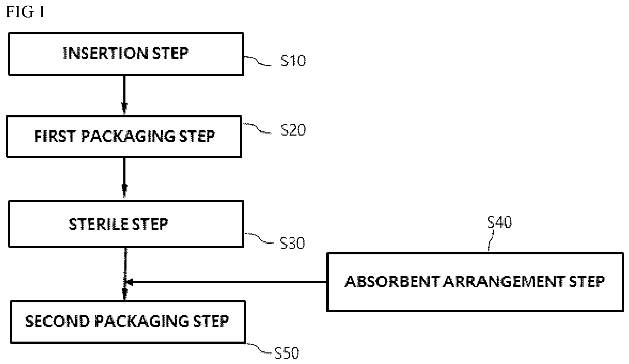

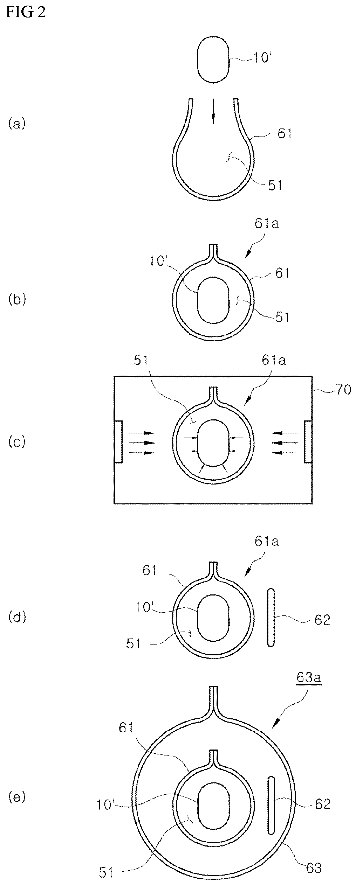

FIGS. 1 and 2 are flowcharts for schematically illustrating a sterilization packaging method for a body-attachable-type biometric monitoring device according to an operation flow according to an embodiment of the present disclosure.

A sterilization packaging method for a body-attachable-type biometric monitoring device according to an embodiment of the present disclosure may comprise an insertion step (S10), a first packaging step (S20), a sterile step (S30), and a second packaging step (S50), and may further comprise an absorbent arrangement step (S40) between the sterile step (S30) and the second packaging step (S50).

The insertion step (S10) is performed by inserting a biometric monitoring device (10'), which is a sterilization packaging object product, into a separate storage chamber (51), and, as illustrated in FIG. 2, the storage chamber (51) may be formed by a first wrapper (61) or be formed using a packing container (not shown) of which one side is open.

The first packaging step (S20) is performed by, in a state that the biometric monitoring device (10') is inserted in the storage chamber (51) at the insertion step (S10), sealing the storage chamber (51) by the first wrapper (61) to protect the biometric monitoring device (10') from the outside of the first wrapper (61) and to form a first package (61a). At that time, the first wrapper (61) is formed with or made of gas permeable material so that the sterilization for the biometric monitoring device (10') can be processed at the sterile process (S30).

The sterile step (S30) can be performed in a way of sterilizing the first package (61a) formed through the first packaging step (S20) by a chemical sterilization method. The chemical sterilization method may be applied as a sterilization method using at least one of EO (Ethylene Oxide) gas, CD (Chlorine Dioxide, ClO₂) gas, low temperature plasma or vapor. Such a chemical sterilization method performs a sterile process for the storage chamber (51) of the biometric monitoring device (10') by exposing the first package (61a) into the inside of a sterilization processing device (70) so that gas for sterilization can be permeated into an inside space (storage chamber) of the first package (61a). Accordingly, the first wrapper (61) wrapping the biometric monitoring device (10') is made of gas permeable material so that the gas for the sterilization can permeate the first wrapper (61), and the storage chamber (51) is sealed so that substance other than gas cannot penetrate it.

The second packaging step (S50) is performed by sealing an external or outside portion or space of the first package (61a), sterilized at the sterile step (S30), by a second wrapper (63) to protect the first package (61a) from the outside of the second wrapper (63) and to form a second package (63a). The second wrapper (63) may be formed with gas non-permeable material having the characteristics of gas non-permeability, and for example, is formed with gas non-permeable material such that a metal coat layer is formed on one side of the second wrapper (63).

If the biometric monitoring device (10') sealed by the first wrapper (61) through the first packaging step (S20) is distributed in a state of the first package (61a), the biometric monitoring device (10') can be contaminated because contaminants such as various types of gas existed in external environment permeate the first wrapper (61) and penetrate into the inside of the storage chamber (51), and, specially, if moisture is permeated, the biometric monitoring device (10') can be damaged by the moisture.

The first wrapper (61) is formed with gas permeable material for a sterilization processing, and therefore, by sealing an external or outside portion or space of the first package (61a) after performing the first packaging step (S20) and the sterile step (S30), contaminated gas, moisture and the like from the outside cannot permeate into the inside space so that the biometric monitoring device (10') can be safely stored in a completely sealed and blocked from the outside.

Accordingly, the biometric monitoring device (10') is sterilized in a state first-packaged by the first wrapper (61) of the gas permeable material, and is second-packaged by the second wrapper (63) of the non-permeable material in a state that the sterilization process is completed, and therefore the biometric monitoring device (10') can be safely stored in a state completely isolated or blocked from the outside.

In a conventional sterilization packaging method, a first packaging process can be omitted and the processes of sterilizing the biometric monitoring device (10') itself and then sealingly packaging the biometric monitoring device (10') with the non-permeable material can be performed, but during the process of packaging the sterilized biometric monitoring device (10') separately, there may be a problem in that the contamination caused by the contaminants can occur. Additionally, when the sterilization process is performed and distributed after packaging the biometric monitoring device (10') with a wrapper of gas permeable material, outside contaminated gas and so on can permeate the wrapper and penetrate into the inside space, and therefore it can cause the damage to the biometric monitoring device (10').

However, the sterilization packaging method according to an embodiment of the present disclosure can perform a first packaging step and a second packaging step separately, can perform sterilization in a state of being first-packaged, and can perform second-packaging in a state of being first-packaged and sterilized, and therefore the penetration of the outside contamination substance which may occur during the sterilization and packaging processes can be prevented and the damage to the biometric monitoring device (10') can be avoided, and the sterilization packaging of the biometric monitoring device (10') can be performed in a convenient and simple way.

Meanwhile, the sterilization packaging method according to an embodiment of the present disclosure can further comprise the absorbent arrangement step (S40) between the sterile step (S30) and the second packaging step (S50).

The absorbent arrangement step (S40) can be performed by arranging an absorbent (62) on an outside surface of the first wrapper (61) after the sterile step (S30), and may be a dehumidifying agent absorbing moisture. The second packaging step (S50) can be performed by forming the second package (63a) in a form of accommodating the absorbent (62) arranged in the inside space of the second package (63a) through the absorbent arrangement step (S40).

Such a absorbent (62) is generally arranged at the inside space of the storage chamber (51) to remove the moisture existed in the inside of the storage chamber (51), but, if the sterilization process is performed in a state that the absorbent (62) is arranged in the inside of the storage chamber (51) as in the conventional way, the gas for sterilization is permeated into the absorbent (62) and therefore the absorbing ability (dehumidification ability) of the absorbent (62) may be significantly lowered. Accordingly, in an embodiment of the present disclosure, after performing the first packaging step and the sterile step, the absorbent (62) is arranged on the outside of the first wrapper (61a), and, specially, because the first wrapper (610 is gas permeable material, even though the absorbent (62) is located at the outside of the first wrapper (61), the absorbent (62) can absorb the moisture from the inside of the first wrapper (61) of the storage chamber (51) thereby performing a stable absorbing function (moisture removal function).

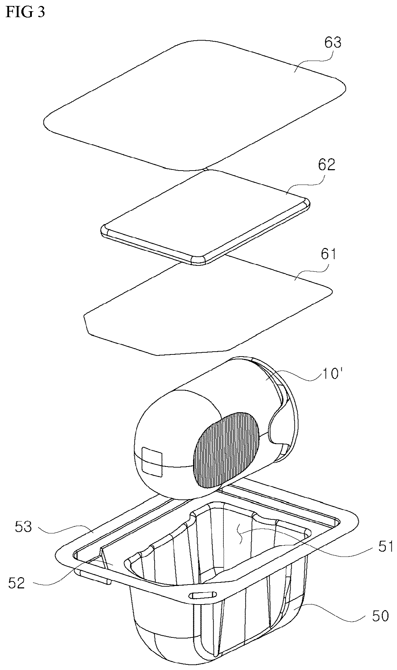

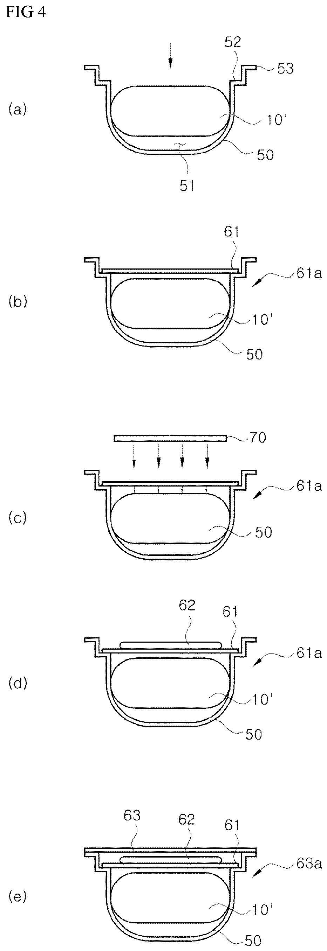

A specific way of applying the sterilization packaging method described above to a continuous blood glucose monitoring device according to an embodiment of the present disclosure will be described in an exemplified way with reference to FIGS. 3 and 4.

FIG. 3 is an exploded perspective view of schematically illustrating a sterilization package structure of a continuous blood glucose monitoring device according to an embodiment of the present disclosure, and FIG. 4 is figures for schematically illustrating a sterilization packaging method for a continuous blood glucose monitoring device according to an operation flow according to an embodiment of the present disclosure.

Firstly, the biometric monitoring device (10') is used as a continuous blood glucose monitoring device, and the storage chamber (51) into which the biometric monitoring device (10') is inserted is formed at the inside space of a package case (50) with a container shape of which one side is open.

A fixing protrusion portion can be formed at the inside surface of the package case (50) so that the position of the biometric monitoring device (10') is fixed in the state that the biometric monitoring device (10') is insertedly received. Additionally, as illustrated in FIGS. 3 and 4, a second attachment surface (53) is formed at the outmost edge end portion of the package case (50) toward an open side of the package case (50), and a first attachment surface (52) is formed on a stepped structure along the outmost edge portion at a position away from the second attachment surface (53) in an inward direction. The first attachment surface (52) and the second attachment surfaces (53) are areas formed to be attached to the first wrapper (61) and the second wrapper (63), respectively.

By applying such a package case (50) to the continuous blood glucose monitoring device, the insertion step (S10) is performed in a way of inserting the biometric monitoring device (10') into the storage chamber (51) formed at the package case (50). At that time, the first packaging step (S20) is performed in a way of sealingly coupling the first package (61) to an open side of the package case (50) in a state that the biometric monitoring device (10') is inserted to the storage chamber (51) of the package case (50). The first wrapper (61) seals the storage chamber (51) formed at the package case (50) in a way of attaching the outmost edge of the first wrapper (61) to the first attachment surface (52) of the package case (50).

As described above, the first wrapper (61) is sealed to form the first package (61a) in a state that the biometric monitoring device (10') is inserted to the storage chamber (51) of the package case (50), and the sterile step (S30) is performed by exposing the first package (61a) to the sterilization processing device (70). At the sterile step (S30), the gas for sterilization penetrates the first wrapper (61) and permeates into the storage chamber (51), and the biometric monitoring device (10') stored in the storage chamber (51) is sterilized.

After that, at the absorbent arrangement step (S40), the absorbent (62) is securely arranged on the outer side surface of the first wrapper (61) coupled to the first attachment surface (52) of the package case (50), and at the second packaging step (S50), the second wrapper (63) is sealingly coupled to the second attachment surface (53) of the package case (50). Because the first attachment surface (52) and the second attachment surface (53) are positioned away from each other, there is a space between the first wrapper (61) and the second wrapper (63) attached to the first attachment surface (52) and the second attachment surface (53), respectively, and the absorbent (62) is arranged at that space.

According to the process described above, the sterilization packaging process for the biometric monitoring device (10') can be performed in a convenient and simple way, and additional contamination which can occur during the sterilization process can be prevented and damage caused by moisture and so on after the completion of the packaging can be prevented thereby performing the more secure sterilization packaging.

Hereinafter, configuration of a continuous blood glucose monitoring device which is applied to the biometric monitoring device (10') according to an embodiment of the present disclosure will be described.

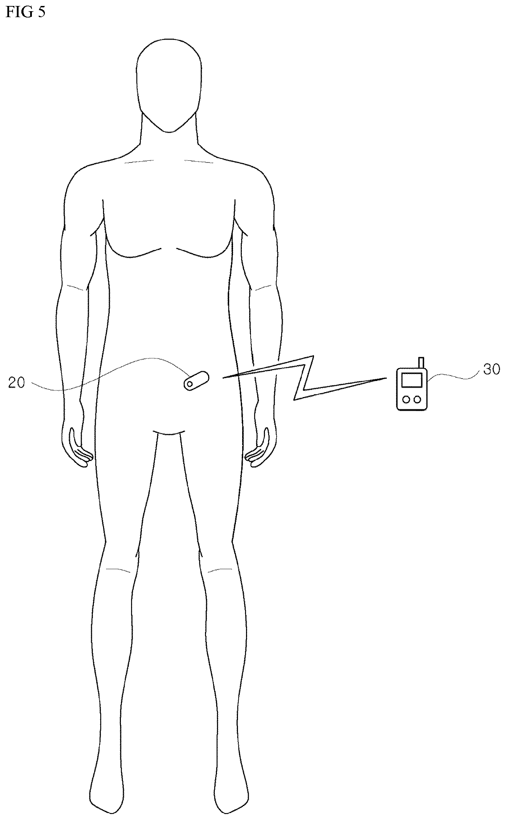

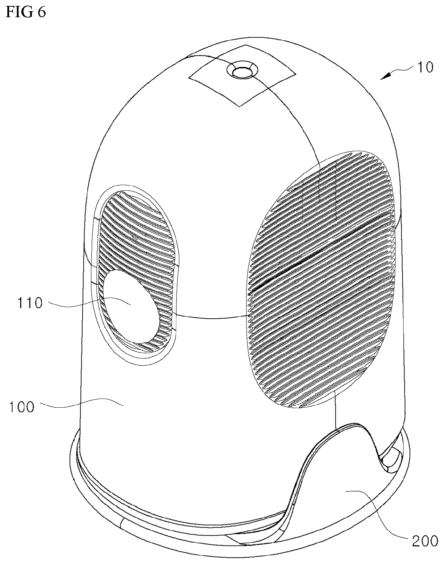

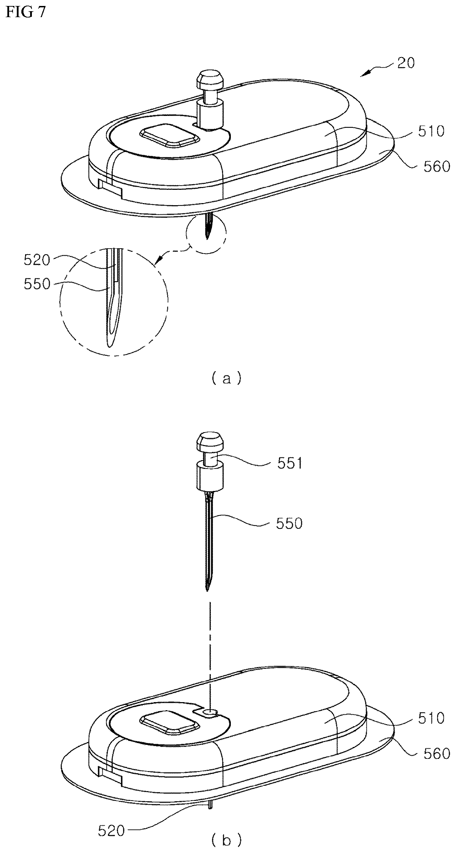

FIG. 5 is a figure for schematically illustrating a basic system of a continuous blood glucose monitoring device according to an embodiment of the present disclosure, FIG. 6 is a figure for schematically illustrating a structure of an applicator of a continuous blood glucose monitoring device according to an embodiment of the present disclosure, and FIG. 7 is a figure for schematically illustrating a configuration of a body attachable unit of a continuous blood glucose monitoring device according to an embodiment of the present disclosure.

A continuous blood glucose monitoring device according to an embodiment of the present disclosure is configured that a body attachable unit (20) including a sensor unit (520) inserted into the human body for continuous blood glucose measurement is configured to be attached to the human body through an applicator (10), the blood glucose can be continuously monitored by insertedly attaching the body attachable unit (20) to the human body by manipulating the applicator (20), and blood glucose information periodically measured by the body attachable unit (20) is transmitted to a terminal (30) to display it.

A continuous blood glucose monitoring device according to an embodiment of the present disclosure may be manufactured as one single unit product in which the body attachable unit (20) is assembled inside the applicator (10), and has a simpler structure which can be easily used by minimizing additional work of a user when using the continuous blood glucose monitoring device.

The body attachable unit (20) may be configured to be attachable to a human body to periodically measure blood sugar level or glucose by extracting body fluid, and transmit the blood glucose measurement result to an external device such as an external terminal (30) and so on. A sensor unit (520) of which one end portion can be inserted into the human body and a wireless communication chip configured to wirelessly communicate with the external terminal (30) can be disposed inside the body attachable unit (20), and therefore, the body attachable unit (20) can be used without additional connection of a separate transmitter.

The applicator (10) is formed such that the body attachable unit (20) is fixedly coupled to inside of the applicator (10), and the applicator (10) is configured to outwardly discharge the body attachable unit (20) according to the manipulation of the user.

In this embodiment, the body attachable unit (20) is assembled and produced in a state that the body attachable unit (20) is inserted into the inside of the applicator (10), and is configured to move in an outward discharge direction pursuant to the operation of the applicator (10) by the manipulation of the user and be attached to the human body.

Therefore, the sensor applicator assembly according to an embodiment of the present disclosure is assembled and manufactured in a state that the body attachable unit (20) is inserted in the inside of the applicator (10) at the manufacturing stage and the body attachable unit (20) can be attached to a skin by only the operation of the applicator (10), and because the sensor applicator assembly is supplied to the user in this state, the user can easily attach the body attachable unit (20) to the skin by only the manipulation simply activating the applicator (10) without extra additional operation for attaching the body attachable unit (20) to the skin. Specifically, since the body attachable unit (20) has the wireless communication chip, no connection with an extra transmitter is needed and therefore it can be used more conveniently.

In a conventional continuous blood glucose measurement apparatus, after removing a body attachable unit, which is separately packaged, precisely inserting it into an applicator, and then operating the applicator, the body attachable unit is attached to a skin, but the work precisely inserting the body attachable unit into the applicator is cumbersome as well as difficult and there is a problem in lowering the accuracy of blood glucose measurement because of contaminating the body attachable unit when young children or elderly adults perform this procedure.

In an embodiment of the present disclosure, at the manufacturing stage, it is manufactured and distributed in a state that the body attachable unit (20) is inserted in the applicator (10), and therefore the step that the user removes the body attachable unit (20) from the package and inserts it into the applicator (10) may be omitted, because the body attachable unit (20) can be attached to the skin by simply manipulating the applicator (10), the usability may be significantly improved, and specifically, the accuracy of blood glucose measurement may be improved by preventing the contamination of the body attachable unit (20).

A separate and additional protection cap (200) can be separably coupled to the applicator (10) in order to block external exposure in a state that the applicator (10) is inserted in the inside of the applicator (10), and it may be configured that the user can attach the body attachable unit (20) to the human body by manipulating the applicator (10) only after the protection cap (200) is separated.

In the embodiment of the present disclosure, an adhesive tape (560) is provided at a side of the body attachable unit (20) contacting the human body to be attached to the body, to protect the adhesive tape (560) a release paper (not shown) is attached to a surface of the adhesive tape (560) contacting the human body, and the release paper of the adhesive tape (560) may be configured to be separated and removed from the adhesive tape (560) during the operation of separating the protection cap (200) from the applicator (10).

For example, the release paper of the adhesive tape (560) may be configured to adhere one side of the release paper to the protection cap (200), and therefore, if the user separates the protection cap (200) from the applicator (10), the release paper (560) may be separated and removed from the adhesive tape (560) together with the protection cap (200). Accordingly, if the user separates the protection cap (200), the release paper of the adhesive tape (560) is separated and removed, and therefore in this status the body attachable unit (20) can be attached to the human body by the operation of the applicator (10).

Additionally, in a state that the body attachable unit (20) is inserted in the inside, the applicator (10) fixes the body the attachable unit (20), and in a state that the body attachable unit (20) is outwardly discharged and moved, the applicator (10) is configured to release the fixed state of the body attachable unit (20). Accordingly, in a state that the body attachable unit (20) is assembled to be inserted in the inside of the applicator (10), the body attachable unit (20) maintains the fixed state, and when the body attachable unit (20) is externally discharged and attached to the skin by actuating the applicator (10), the state fixed between the applicator (10) and the body attachable unit (20) is released, and therefore if the applicator (10) is separated in this state the applicator (10) is separated from the body attachable unit (20) and only the body attachable unit (20) remains on the skin.

Meanwhile, the body attachable unit (20) according to an embodiment of the present disclosure is configured to cause the sensor unit (520) and the wireless communication chip to initiate their operations through a separate switching means controlled by the user. Accordingly, after inserting and attaching the body attachable unit (20) to the human body by using the applicator (10), the user may initiate to operate the body attachable unit (20) through the switching means or other appropriate means included in the body attachable unit (20), and from the time of the initiation of the operation the sensor unit (520) and the wireless communication chip may be operated, the blood glucose of the human body may be measured, and the measurement result may be transmitted to the external terminal (30). In this embodiment, the switching means operated by the user may be implemented in various ways.

Additionally, in the body attachable unit (20), the sensor unit (520) is disposed in a housing (510), and one end portion of the sensor unit (520) outwardly protrudes from the housing (510) so that it can be inserted and attached to the human body. The sensor unit (520) may comprise a sensor probe unit to be inserted into the human body, and a sensor body unit disposed inside the housing (510), and the sensor probe and the sensor body unit are formed as one end portion and another end portion of the sensor unit (520), respectively, and in a bent shape.

In this embodiment, in order to smoothly perform the body insertion process of the sensor unit (520), an insertion guide needle (550) may be separatably coupled to the housing (510). The insertion guide needle (550) may surround one end portion of the sensor unit (520) and be configured to be inserted together with the sensor unit (520) into the human body so that one end portion of the sensor unit (520) can be stably inserted into the human body.

As shown in FIG. 7, the insertion guide needle (550) may be separatably coupled to the housing (510) in a direction penetrating the top and bottom of the housing (510) of the body attachable unit (20), the insertion guide needle (550) may be formed to have a structure covering the outside of the sensor unit (520), and a need head (551) is formed at the upper end portion of the insertion guide needle (550). If the body attachable unit (20) is moved in the direction outwardly discharged by the applicator (10), the insertion guide needle (550) is inserted into the human body first before the sensor unit (520) is inserted into the human body and the insertion guide needle (550) may support the sensor unit (520) such that the sensor unit (520) can be stably inserted in the skin. The insertion guide needle (550) may be coupled with a needle extracting body (not shown) of the applicator (10) through the needle head (551), and after the body attachable unit (20) is inserted and attached to the human body by the operation of the applicator (10), the insertion guide needle (550) may be configured to be extracted and removed from the human body by the needle extracting body (400) of the applicator (10).

After the insertion guide needle (550) is extracted and removed from the human body, a state that the body attachable unit (20) is insertedly attached to the human body is maintained, and the sensor unit (520) of the body attachable unit (20) measures blood glucose information from body fluid in a state that its one end portion is inserted to the skin. For this blood glucose information measurement, a plurality of electrode layers are formed at the sensor unit (520), and each electrode layer is formed to be connected to an electric contact point of an external electronic device.

The foregoing descriptions have been presented in order to explain certain principles of the present disclosure by way of example, and a person having ordinary skill in the art which the present disclosure relates could make various modifications and variations without departing from the essential features of the present disclosure. Accordingly, the foregoing embodiments disclosed in the present disclosure shall be interpreted as being illustrative, while not being limitative, of the principle and scope of the present disclosure. It should be understood that the scope of the present disclosure shall be defined by the Claims and all of their equivalents fall within the scope of the present disclosure.

## Claims

1. A sterilization packing method for a body-attachable-type biometric monitoring device, the method comprising:
an insertion step of inserting the body-attachable-type biometric monitoring device into a storage chamber;
a first packaging step of forming a first package by sealing the storage chamber by a first wrapper of gas permeable material in a state that the body-attachable-type biometric monitoring device is inserted in the storage chamber;
a sterile step of sterilizing the first package formed through the first packaging step by a chemical sterilization process; and
a second packaging step of forming a second package by sealing an outside of the first package by a second wrapper,
wherein the second wrapper comprises non-permeable material having gas non-permeable character.

2. The sterilization packing method for a body-attachable-type biometric monitoring device according to claim 1, further comprising an absorbent arrangement step of arranging an absorbent at an outside surface of the first wrapper after the sterile step,
wherein the second packaging step comprises forming the second package in a form of accommodating the absorbent, arranged through the absorbent arrangement step, in an inside of the second wrapper.

3. The sterilization packing method for a body-attachable-type biometric monitoring device according to claim 2, wherein:
the storage chamber is formed at an inside space of a package case with a container shape of which one side is open, and
the first packaging step comprises sealingly coupling the first wrapper to the open one side of the package case in a state that the body-attachable-type biometric monitoring device is inserted in the storage chamber of the package case.

4. The sterilization packing method for a body-attachable-type biometric monitoring device according to claim 3, wherein:
a second attachment surface is formed at an outmost edge portion of the package case facing the open one side,
a first attachment surface is formed along an outer edge of a stepped structure at a position away from the second attachment surface in an inside direction of the package case, and
at the first packaging step, an outmost edge portion of the first wrapper is sealingly coupled to the first attachment surface.

5. The sterilization packing method for a body-attachable-type biometric monitoring device according to claim 4, wherein, at the second packaging step, the second wrapper is sealingly coupled to the second attachment surface.

6. The sterilization packing method for a body-attachable-type biometric monitoring device according to claim 5, wherein, at the absorbent arrangement step, the absorbent is arranged on an outside surface of the first wrapper coupled to the first attachment surface of the package case.

7. The sterilization packing method for a body-attachable-type biometric monitoring device according to any one of claims 1 to 6, wherein the body-attachable-type biometric monitoring device is configured to be attachable to a human body and periodically measure blood glucose.

8. The sterilization packing method for a body-attachable-type biometric monitoring device according to any one of claims 1 to 6, wherein, at the sterile step, the chemical sterilization process is a sterilization method using at least one of EO gas, CD gas, low temperature plasma, and steam.

9. The sterilization packing method for a body-attachable-type biometric monitoring device according to any one of claims 1 to 6, wherein the second wrapper comprises gas non-permeable material of which one surface is coated with a metal coating layer.

10. A body-attachable-type biometric monitoring device sterilizingly packaged by the sterilization packing method according to any one of claims 1 to 6.
